# EUROPEAN PATENT APPLICATION

(11) **EP 2 746 386 A1**
(43) Date of publication of application: **25.06.2014**
(21) Application number: 12198932.1
(22) Date of filing: 21.12.2012
(51) Int. Cl.: C12N 5/071

(54) **Materials and methods for cell culture**

(71) Applicant: Lonza Cologne GmbH, 50829 Köln (DE)
(72) Inventor: van den Bos, Christian, 48268 Greven (DE); Bossie, Alexis, North Potomac, MD 20878 (US); Brill, Silke, 50825 Köln (DE); Safavinab, Agnieszka, 53844 Troisdorf (DE); Jüngerkes, Frank, 41470 Neuss (DE); Reinisch, Barbara, 51375 Leverkusen (DE); Rosenbaum, Claudia, 40591 Düsseldorf (DE); Schenk, Judith, 50678 Köln (DE)
(74) Representative: Remus, Alvaro Johannes

(57) **Abstract**

Methods and compositions for improved cell growth and serum-reduced or -free cell culture media.

## Description

### FIELD

Methods and compositions for culturing cells are disclosed. More specifically, compositions for improved media used in mesenchymal stem cell (MSC) culture, methods of culturing mesenchymal stem cells, and cells produced from such methods and compositions are described.

### BACKGROUND

Adult mesenchymal stem cells (MSCs) can be isolated from a variety of organs and tissues, including brain, bone marrow, peripheral blood, blood vessels, umbilical cord, adipose tissues, skeletal muscle, skin, dental pulp, heart, gut, liver, ovarian epithelium, and testis. They are thought to reside in a specific area of each tissue (called a "stem cell niche"). Stem cells may remain quiescent (non-dividing) for long periods of time until they are activated by a normal need for more cells to maintain tissues, or by disease or tissue injury.

MSCs are gaining increasing interest as a potential source for therapeutic approaches in in regenerative medicine. Stem cells can self-renew and have the capacity to differentiate into specific cell types or can be therapeutically used for the production and secretion of soluble factors that will promote tissue regeneration.

Stem cell treatments are a type of intervention strategy that introduces adult stem cells into damaged tissue in order to treat disease or injury. Some examples of potential treatments include regenerating bone using cells derived from bone marrow stroma, developing insulin-producing cells for type 1 diabetes, and repairing damaged tissue such as heart muscle following a heart attack, brain tissue following stroke or in Parkinson Disease, skin replacements MSCs have been employed for replacement of bone marrow, skin, heart, and in corneal transplantation, graft versus host disease, hepatic and renal failure, lung injury, multiple sclerosis, rheumatoid arthritis, diabetes and lupus diseases etc.

In aging western countries there is a growing demand for the use of stem cells as therapeutic treatments. Critical to these treatments is the ability to grow and expand stem cells in order to provide enough cell material for Regenerative Medicine and for producing an affordable therapy product for patients. Typically, there is a very small number of stem cells in each tissue, and once removed from the body, their capacity to divide is limited, making generation of large quantities of stem cells difficult. Scientists in many laboratories are trying to find better ways to produce large quantities of adult mesenchymal stem cells for the growing demand in treating injury or disease. Therefore, new and improved methods of stem cell expansion have utility in cell therapy treatments.

Mesenchymal stem cells (MSCs; also called bone marrow stromal stem cells) occur at low frequency in tissues and must be expanded in vitro to obtain sufficient numbers for research and therapeutic applications. The majority of modem culture techniques still take colony-forming unit-fibroblasts (CFU-F) approach, where raw unpurified bone marrow or ficoll-purified bone marrow mononuclear cells are plated directly into cell culture plates or flasks. Most of the currently used therapeutic cells are defined by their adherence to tissue culture plastic and they are propagated in anchorage/attachment-dependent fashion. This is also true for potential alternatives such as embryonic stem cells (ES cells) and induced pluripotent stem cells (iPS). Such propagation is inherently limed with severe limitations in scale and economy. For cell culture expansion, cells are typically seeded onto planar surfaces to which they attach, left to proliferate and harvested by means of proteolytic enzymes and /or calcium removal.

Human mesenchymal stem cells (hMSCs) grow well on planar surfaces such as found in T-flasks. Scale-up in production can then either occur through scale-out technology, meaning multiplying the number of flasks or, more likely, by scale up version of small flasks to larger containers. Just increasing the vessel size rapidly reaches its limitations where large containers cannot be handled manually anymore. Furthermore this strategy is becoming uneconomical due to the requirement of high amounts of expensive media and serum. Alternatively, attachment dependent cells can be produced in large quantities in bioreactors on microcarriers, which will increase the surface area on which the cells can be grown, while culturing the cells under suspension condition with less media requirement. However, cell yield drops drastically when cells are cultured in suspension conditions. It therefore would be beneficial to develop a method of growing cells in suspension conditions that overcomes the loss of cell yield.

Another issue in human stem cell production for therapeutic markets is the desire to avoid using animal derived products, prominently serum (fetal bovine serum, FBS). Although currently the use of bovine serum remains permissible from a regulatory point of view, there is a clear rationale to move to serum free/products-of animal-origin-free culture systems. Part of his rationale is the avoidance of possible pathogens that might be present in material derived from animal origin as well as the limited availability of the product, hence economic considerations. Furthermore production under chemically defined procedures should be a high priority for therapeutic products. FBS contains a high variability of unknown factors with varying concentrations in between individual FBS lots; hence production of therapeutic cells under serum-reduced or serum-free conditions is desirable. The factors present in serum may present risks of biological contamination since such materials are extracted from animal tissues, and the variable or undefined structures of such materials may lead to variability in cell yields. To improve safety and reliability, and reduce costs of cell therapies, there remains a need for a developing a more closely chemically-defined cell culture medium, with reduced serum content or even free of serum that promotes expansion of mesenchymal stem cell populations while maintaining a pluripotent phenotype.

Serum is not only a very expensive and cost driving factor in the production of stem cells, but also includes the risk of transferring animal-borne pathogens.

Therefore, there remains a need for effective methods to improve cell culture growth and striving towards serum free media.

### SUMMARY OF THE INVENTION

The present disclosure provides media, methods, and kits useful in culturing, expanding and cryopreserving stem cells.

A cell culture medium is provided, the cell culture medium comprising a base medium, a microcarrier, and, optionally, a media supplement. In another aspect, the disclosure provides for a cell culture medium comprising a base medium and, optionally, a media supplement.

In a preferred embodiment, the micocarrier is selected such that its surface structure and/or chemistry provide features missing in a particular medium. Preferably, the microcarrier may be selected to provide an adhesion surface for an adult human mesenchymal stem cell. In one embodiment, the microcarrier is coated with at least one adhesion molecule.

A cryopreservation medium is provided, the cryopreservation media comprising a base cryopreservation medium, a base media and a media supplement.

A method for expanding stem cells is also provided, the method comprising growing the cells in a cell culture medium according to the invention comprising a base medium, a microcarrier, and, optionally, a media supplement. In this method, the stem cells preferably are human mesenchymal stem cells. The human mesenchymal stem cells may be expanded in suspension in a serum-free, essentially serum free, or serum reduced culture medium, the culture medium comprising: a media base selected from the group consisting of X-VIVO 15 and X-VIVO 20; a microcarrier; and optionally, a media supplement selected from the group consisting of MSCGM-CD SingleQuot.

A method for cryopreserving stem cells is also provided, the method freezing the cells in a cryopreservation media, comprising a base medium, and, optionally, a media supplement.

A stem cell culture is also provided, the stem cell culture being obtained by growing a stem cell in a cell culture medium comprising a base medium, a microcarrier, and, optionally, a media supplement.

A kit for growing stem cells is also provided, the kit comprising a base medium, and, optionally, a media supplement. Preferably, the kit further comprises a microcarrier. In another preferred embodiment, the kit includes base medium, microcarrier, and media supplement as individual components, as pre-mixes with one or more of the other components of the kits, or as a premixed cell culture medium.

A kit for cryopreserving stem cells is also provided, the kit comprising a cryopreservation medium, a base medium, a microcarrier, and, optionally, a media supplement. In another aspect the microcarrier can be omitted in the kit.

Other aspects and embodiments will be apparent in light of the following description, examples, and figures.

### BRIEF DESCRIPTION OF THE FIGURES

Figure 1 shows the variety of media compositions tested.

Figure 2 shows the growth of mesenchymal stem cells in a variety of culture media.

Figure 3 shows data supporting the conclusion that new combination of media components supports better growth on planar surfaces compared to the so far called optimized standard conditions.

Figure 4 shows results from "weaning off' experiments in which serum content was reduced.

Figure 5 shows the results of culturing stem cells in bioreactors with microcarriers and the new media composition not only allows serum reduction but shows a 60% increase in cell yield per used ml media.

Figure 6 shows cryopreservation of cells in X-VIVO-containing media.

Figure 7 shows a comparison of growth of hBMMSC in planar culture in X-VIVO20-MCD media with 5 % serum compared to DMEM with 10% serum.

### DETAILED DESCRIPTION

The present disclosure provides media, methods, and kits useful in culturing, expanding and cryopreserving stem cells.

In an aspect, the medium comprises a base medium, a media supplement, and optionally, a microcarrier.

In another aspect, the medium comprises a base medium and optionally a media supplement.

As used herein, the term "base medium" refers to liquid medium used for the processes according to the invention comprising a suitable carbon source such as a sugar, lipids, vitamins and amino acids and a buffering system. The medium contains levels and/or ratios of salts and nutrients needed to support MSC expansion and vitality. The medium may be in dry or liquid form, and may be prepared from a plurality of separate stock compositions (e.g., each independently existing in dry or solution form) that can be combined prior to use. For example, the medium may be prepared from two, three, four, or more stock compositions (each independently in dry or solution form), and where necessary mixed with aqueous diluent prior to use to give a 1X medium formulation. In an aspect, Standard media, for example, can include MSCGM-CD or X-VIVO™15, X-VIVO™20 (Lonza, Walkersville, MD), DMEM, EAGLE, and alphaMEM.

In an aspect, the base medium is a source for the salts and nutrients needed to support MSC expansion and vitality, including amino acids, vitamins, and various inorganic salts. The basal medium may be, for example, X-VIVO™ 15 , X-VIVO™ 20, Iscove's modified Dulbecco's medium (IMDM), Dulbecco's Modified Eagle Medium (DMEM), Ham's F-10, Ham's F-12, MCDB series, RPMI 1640, Minimum Essential Medium (MEM), alpha MEM, MSCGM-CD, MSCGM, MesenCult®-XF, MesenPRO , StemPro®, EGM2MV, Fischer's, Glascow Minimum Essential Medium (GMEM), L-15 (Leibovitz), McCoy's 5A Modified, William's Medium. Other such basal media might be used as a blend of Dulbecco's modified Eagle's Medium and Ham's-F12 medium at a 1:1 ratio. The requirements of the basal medium are to provide i) inorganic salts so as to maintain cell osmolality and mineral requirements (e.g., potassium, calcium, phosphate, etc.), ii) essential amino acids required for cell growth, that is, amino acids not made by endogenous cellular metabolism, iii) a carbon source which can be utilized for cellular energy metabolism, typically glucose, and iv) various vitamins and co-factors, such as riboflavin, nicotinamide, folic acid, choline, biotin, and the like, as my be required to sustain cell growth and viability. The medium may also provide a buffer system to maintain the pH of the medium during cultivation of cells as well as a source of glutamine.

By way of example and not limitation, the base medium is selected from the group consisting of X-VIVO™ 10, X-VIVO™ 15, and X-VIVO™ 20. X-VIVO™ 10, X-VIVO™ 15, and X-VIVO™ 20 are chemically defined, serum-free hematopoietic cell media that provide nutritionally complete and balanced environments for a variety of cells including lymphokine activated killer (LAK) cells, peripheral blood lymphocytes (PBL), and tumor infiltrating lymphocytes (TIL). These media do not contain any exogenous growth factors, artificial stimulators of cellular proliferation, or undefined supplements. They are devoid of any protein kinase C stimulators and are suitable for the investigation of second messenger systems in the activation of human and murine lymphocytes. The formulations are complete and contain pharmaceutical grade human albumin, recombinant human insulin, and pasteurized human transferrin.

The X-VIVO™ 10 Media formulations are designed to support the generation of LAK cells in a serum-free environment. The original protocols involved the incubation of patient or normal donor peripheral blood lymphocytes (PBL) at 1.0 - 3.0 × 10⁶ cells/ml for a period of 3 days in the presence of 1,000 Cetus units of rIL-2/ml. Optimal LAK cell generation is achieved when peripheral blood lymphocytes are incubated for 3-10 days at a density of 1.0 - 6.0 × 10⁶ cells/ml in the presence of 100 - 1,000 Cetus units of rIL-2.

X-VIVO™ 15 is optimized for the proliferation of Tumor Infiltrating Lymphocytes (TIL) under serum-free conditions. X-VIVO™ 15 Media are similar in composition to X-VIVO™ 10 Media and have been optimized for the proliferation of tumor infiltrating lymphocytes (TIL) under serum-free conditions. X-VIVO™ 15 Media support the proliferation of purified CD3+ cells isolated from peripheral blood and human tumors. X-VIVO™ 15 Media can also be used to support the growth of human monocytes, macrophage cells and cell lines, PBL, granulocytes, and natural killer (NK) cells. In addition, X-VIVO™ 15 Media provide a serum-free environment for the expansion of HUT-78 and related human lymphocytic cell lines.

X-VIVO™ 20 Medium is optimized to support the generation of lymphokine activated killer (LAK) cells from monocyte depleted peripheral blood lymphocytes (PBL) at high density. Initial cell densities between 2.0 - 3.0 × 10⁷ cells/ ml can be used to successfully generate LAK cells. X-VIVO™ 20 Medium has also been shown to be useful as a growth medium for PBL and tumor infiltrating lymphocytes (TIL).

As used herein, the term "microcarrier" shall refer to particles providing attachment and growth surfaces for cells that have to be propagated in anchorage/attachment-dependent fashion, hence allowing expansion of anchorage-depending stem cell cultures under suspension conditions. Microcarrier cultures allow a high yield stem cell expansion in various culture volumes providing a method for scale-up of stem cell production.

In an aspect, the microcarrier is selected from the group consisting of commercially available microcarriers, such as Cytodex ™ (GE Healthcare, GB), Hillex (Solo Hill, USA) or the like, including but not limited to temperature-controlled microcarriers from which the cells are removed by a change of temperature, preferably by lowering the temperature. Microcarriers can be selected from the group made of different materials such as plastic, glass, gel-like materials, and metals. Microcarriers can be particles with electrically charged or uncharged surfaces, or particles that have an unporous or porous structure which allows the cells to attach in smaller niches. Microcarriers may be coated or treated with chemical or biological molecules such as collagen, polysaccharides, peptides, antibodies etc. Microcarriers that are coated with specific molecules are commercially available such as ProNectin F microcarriers. In addition, there are commercially available treatments for microcarriers such as CELLstart™( Life Technologies), MesenCult™-XF Attachment Substrate (Stemcell), collagen, fibronectin or proncetin F. Microcarriers can be selected from the group of microcarriers comprising CYTODEX 1™, CYTODEX 3™ (GE Healthcare), HILLEX II™, HILLEX ™ (SOLO HILL), PLASTIC, GLASS, PRONECTIN F (SIGMA), CULTISPHER (PERCELL BIOLYTICA).

In an aspect, the microcarrier comprises HILLEX II™, CYTODEX 3™, and/or a plastic microcarrier.

In an aspect, a micocarrier is selected such that its surface structure and/or chemistry provide features missing in a particular medium.

For example, serum provides several functions including adhesion molecules. Cell adhesion is not directly to a plastic surface but through mediators such as adhesion proteins. Hence, in a serum-free culture the microcarrier surface may provide an alternative by being coated with said adhesion molecules. Adhesion is but one example for such functional complementation and hence several microcarriers selected for their perceived approvability were tested.

In an aspect, the microcarrier is selected to provide a function selected from the group consisting of physical support, signaling that adhesion has occurred, influencing shape, survival, and metabolism of the cells

In an aspect, the microcarrier is selected to provide an adhesion surface for an adult human mesenchymal stem cell, such as by providing a microcarrier coated with at least one adhesion molecule. Adhesion molecules can be selected from the group of extracellular matrix peptides, fibronectin, laminin, pronectin etc.

As used herein, the phrase "media supplement" shall refer to media additives that confer growth/proliferation and viability benefits *in vitro.* The media supplements provide factors further supporting or enhancing nutrition, attachment and/or growth of stem cells while maintaining their pluripotent characteristics.

In an aspect, the media supplement comprises at least one component selected from the group consisting of MSCGM-CD™ SingleQuots®, FGM-CD™ SingleQuot™" EGM-2 SingleQuot Kit Suppl. & Growth Factors, MesenCult™ Mesenchymal Stem Cell Stimulatory Supplements (Human), and StemPro® MSC SFM. In a further aspect, the media supplement is selected from the group consisting of MSCGM-CD™ SingleQuots®. In an aspect, the media supplement is MSCGM the commercially available MSCGM-CD™ SingleQuots®.

In an aspect, the medium is a serum free medium, essentially serum free medium, or serum reduced medium. In an aspect, the term "serum free" shall refer to media containing no serum. As used herein, the term "essentially serum free" shall refer to a medium containing an insufficient amount of serum to affect cell growth characteristics. As used herein, the phrase "serum reduced" shall refer to a chemically defined medium equal to or less than 10% (v/v) serum, from about 5% and about 10% (v/v) serum, from about 2% and about 5% (v/v) serum, from about .1% and 2% (v/v) serum, and from 0 and from about 2% (v/v) serum. In an aspect, the medium comprises not more than 2% (v/v) or even lower % (v/v) serum.

In an aspect, the medium comprises: a media base selected from the group consisting of X-VIV™15 and X-VIVO™20; a microcarrier selected from the group consisting of Hillex II; and optionally a media supplement selected from the group consisting of MSCGM-CD™ SingleQuots®. In another aspect the microcarrier can be omitted.

The medium may comprise additional components, such as carbon source(s), pH buffering agents, fatty acids, and surface active agents to support the expansion of MSCs from a source population. The medium may contain carbon sources suitable for use by MSCs such as dextrose, pyruvic acid, putrescine, malic acid, lecithin, and/or ethanolamine. The medium may comprise one or more pH buffering agents, including HEPES Buffer and/or sodium bicarbonate. The pH of the medium is generally in the range of about 6.6 to about 7.4. Other buffering agents may be used that are compatible at these pH ranges. In addition the medium may comprise additional, more stable glutamine sources such as GlutaMAX^{™} (Life Technologies).

As used herein, the term "cryoprotection" refers to freezing the cells for storage by preserving their viability. In an aspect the freezing medium is comprised of components from the group of dimethylsulfoxide (DMSO), DMEM, FBS, X-VIVO™15, X-VIVO™20, MSCGM-CD™ SingleQuots®, PlasmaLyte or Profreeze.

In another aspect, a method of expanding stem cells is provided, the method comprising growing the stem cells in a medium as described herein.

In an aspect, the stem cells are attachment-dependent mesenchymal stem cells. The source cells may be marrow progenitor cells, adipose precursor cells, or mesenchymal stem cells, stem cells or precursor cells derived from umbilical cord blood, dental pulp or induced pluripotent stem cells (iPSCs), for example. As disclosed herein, such cells maintain their potential for differentiation, for example, to adipocytes, chondrocytes, or osteocytes. MSCs expanded with the culture medium of the invention maintain their proliferative capacity and differentiation potential, as compared to, for example, expansion in high serum-containing medium. In another aspect, the stem cells are human mesenchymal stem cells.

In an aspect, the mesenchymal stem cells can be grown and expanded in planar systems such as tissue culture flasks or vessels of different sizes as single or multiple stacks, providing growth areas for attachment-depending cells. These cell culture products allow constant gas exchange while minimizing the risk of contamination, sterile filling and emptying possibilities. In another aspect the cells can be grown on microcarriers in bioreactors for single- or multi-use.

In various embodiments, the cells are cultured in medium designed to promote the proliferation and expansion of mesenchymal stem and/or precursor cells, including for large scale production. For instance, MSCs may be expanded from an inoculum population to yields of greater than about 4 fold changes. Such yields may be obtained after several passages in culture, such as from about 3 to 12 about passages. Thus, such yields may be obtained after 5, 6, 7, or more passages in culture. In another aspect expansion of cells can be assessed under static conditions or under non-static conditions such as stirred, shaking or rocking movements. Growth surface can be enhanced by addition of microcarriers for propagation of cells under suspension conditions.

The cells are generally maintained in culture under standard conditions known for culturing MSCs, such as 37°C, 5% CO2, and 95% humidity. The exact conditions can be adjusted by the skilled artisan as needed. A person of ordinary skill in the art would assess normoxic (20% pO₂ or hypoxic (<5 % pO₂) conditions for stem cell culture derived from different tissues. Stirring, rocking or shaking conditions will be varied to determine optimal conditions for reduction of shear stress levels and turbulences. For optimal mesenchymal stem cell production bioreactor or vessel designs, as well as impeller designs have to be taken into accounts depending on the culture size. Stirring- shaking or rocking speeds and mode (continuous, intermittent, etc.) need to be determined for each individual condition.

In an aspect, human mesenchymal stem cells are expanded in suspension in a serum-free, essentially serum free, or serum reduced culture medium, the culture medium comprising: a media base selected from the group consisting of X-VIV™15 and X-VIVO ™20; a microcarrier; and optionally a media supplement selected from the group consisting of MSCGM-CD Single Quots. In another aspect the microcarrier can be omitted.

In an aspect, a stem cell culture is provided, the stem cell culture being obtained by growing a stem cell in a cell culture medium comprising a base medium, a microcarrier, and, optionally, a media supplement. In an aspect, the cell culture is obtained from a serum-free, essentially serum free, or serum reduced cell culture medium.

A kit for growing stem cells is also provided, the kit comprising a base medium, a microcarrier, a treatment for microcarriers e. g. a coating substrate, and, optionally, a media supplement. The base medium, microcarrier, and media supplement may be provided as individual components, as pre-mixes with one or more of the other components of the kits, or as a premixed cell culture medium. In an aspect, the kit comprises a serum-free, essentially serum free, or serum reduced cell culture medium. The components of the kits may be provided as concentrated component stocks or premixed component stock, as a concentrated cell culture medium, or as a cell culture medium at working concentrations.

As shown in the following examples, the media compositions, methods, and kits allow excellent cell expansion in suspension conditions. The media compositions, methods, and kits allow for reduced or even serum-free conditions for large-scale stem cell expansion.

The following examples are illustrative only. Other aspects and embodiments will be readily apparent in light of the present description, examples, and figures.

### EXAMPLES

### Example 1

hMSCs have not yet been successfully expanded in the absence of attachment surfaces, i.e. full suspension and are typically expanded in planar systems. Tests for expansion of stem cells in suspension with the aid of microcarriers as growth surfaces have been performed on hMSCs grown on different microcarriers in 100ml bioreactor cultures.

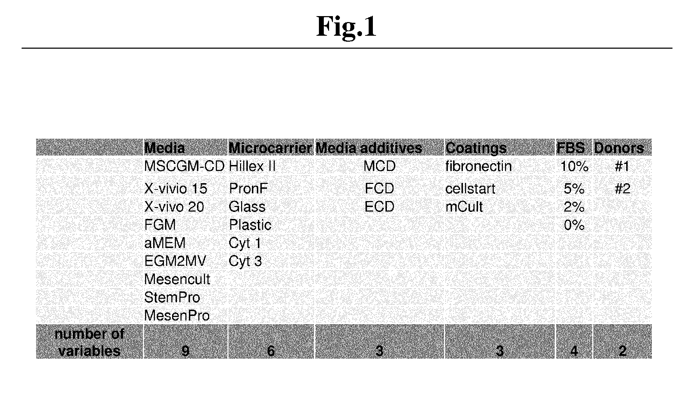

In a first screen with different media and microcarriers, the best performing conditions have been identified. See Figure 1.

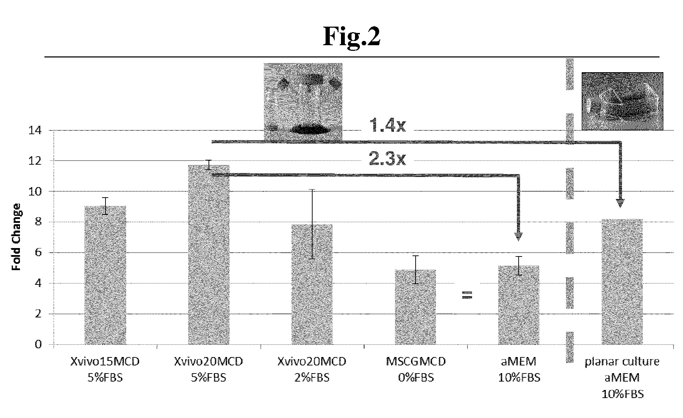

The combination of X-VIVO™15/ X-VIVO™20 media with supplement MSCGM SingleQuot Kit outperformed every other tested medium and has further been evaluated in subpopulations of BMMSCs, hMPCs (human Mesenchymal Precursor Cells) for expansion of stem cells in suspension (Fig. 2). Cells have been inoculated with microcarriers in spinner bioreactors. Subsequently, after a single rest phase of 24 h cultures have been agitated intermittently. Cell yields are depicted as Fold Change (FC); meaning cell number harvested / cell number inoculated - for FC=2 that means 2 Mio cells harvested / 1 Mio cells inoculated.

Results as shown in Figure 2 delineate that the new media combination does not only allow better cell expansion as the optimal condition for planar cultures in alpha MEM 10%FBS (1.4 times better FC) but performs even 2.3 fold better compared to standard media in a bioreactor (alpha MEM10%FBS). In addition, the combination already gives excellent results when serum is reduced to 5% when compared to control conditions (planar cultures and suspension cultures in alpha MEM containing 10% FBS).

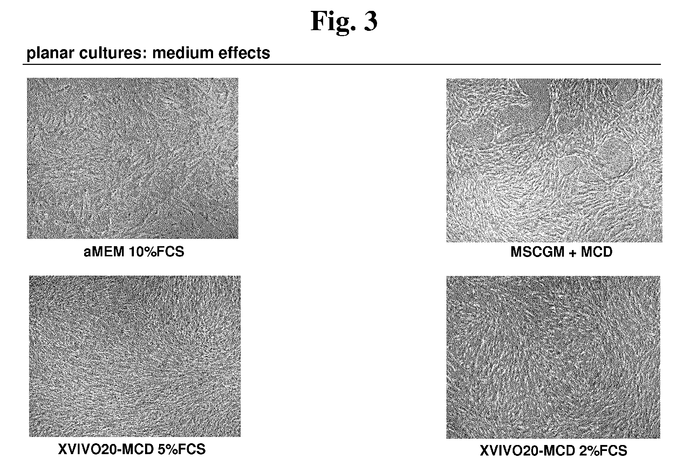

The new combination of media components supports better growth of hMPCs on planar surfaces compared to the conditions commonly referred to as optimized standard conditions (aMEM-10%FBS or MSCGM + CD SingleQuots). The new combination of media components supports better growth on planar surfaces compared to the so far called optimized standard conditions (alphaMEM-10%FBS or X-VIVO20 MSCGM-CD Single Quots with 5% or 2 % serum). See Figure 3.

### Example 2

Media were tested for their ability to promote cell expansion in a spinner system. Tests were in combination with various microcarriers; the rationale being that the surface structure/chemistry of microcarriers might compensate for features missing in a particular medium. Per example, serum provides several functions including adhesion molecules -cell adhesion is not directly to a plastic surface but through mediators such as adhesion proteins. Hence, in a serum-free culture the micro/carrier surface may provide an alternative by being coated with said adhesion molecules. Adhesion is but one example for such functional complementation and hence several microcarriers selected for their perceived approvability were tested.

Media tested include MSCGM-CD, X-VIVO 15, X-VIVO 20, FGM, alphaMEM, EGM2MV, Mesencult, StemPro, and MesenPro. Several combinations of base medium (e.g. here X-VIVO 15, 20, with supplements from other media (here: MSCGM-CD Single Quots) as well as serum additives (e.g. 2% or 5%) were tested.

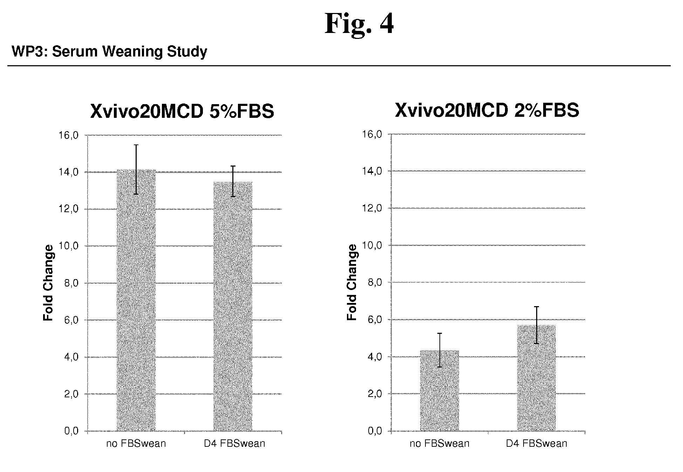

Results indicate that many media tested failed for hMPCs. MSCGM-CD serum/free worked sporadically only. Only the X-VIVO™ 15 and 20 based formulations with the specific additives MSCGM-CD Single Quots plus serum at 2 or 5% did perform acceptably; 5% serum was superior to 2%. Use of this medium reduced usage of serum by correspondingly 50 or 80%. Use of this medium increased growth rates in spinners above those found in planar culture. After an initial phase we were able to exchange 50% of the medium against a medium without serum without observing any ill effects. These weaning off-experiments, where cells first have been grown for 3 passages in standard alphaMEM 10% FBS (as planar cultures) and subsequently were cultured in the new media combination with 10%FBS (X-VIVO20 + MSCGM-CD Single Quots) or in XVIVO20 + MSCGM-CD Single Quots) with only 5% or 2% FBS showed very sufficient Fold Changes for these conditions (Figure 4). These experiments tested new media combination for reducing serum content in cell production.

In prolonged spinner cultures X-VIVO has supported a cell density of up to 500.000 cells per ml, i.e. as much as 5x reported previously. This is the result of a current study and may not be limiting. The final cell yield measured is the result of proliferation minus cell death. While proliferation tends to be in the focus of media work, we considered examining rates of cell death instructive. We compared cells cultured in spinners for up to 12 days in either X-VIVO + supplement (2 and 5% serum) against those cultured in alpha-MEM and examined the combined proportion of dying/dead cells (judged by flow cytometry combining cells positive for apoptosis and permeable for an otherwise excluded dye). Cells cultured in alpha-MEM displayed a proportion of compromised cells of more than 35% whereas cells cultured in X-VIVO + supplement displayed a proportion of compromised cells of less than 15%. Thus, the observed beneficial effect of X-VIVO + supplement compared to alpha-MEM may be due only partially to increased proliferation rates substantially affected by a reduced rate of cell death. Use of this medium also greatly improved the growth rate in planar culture.

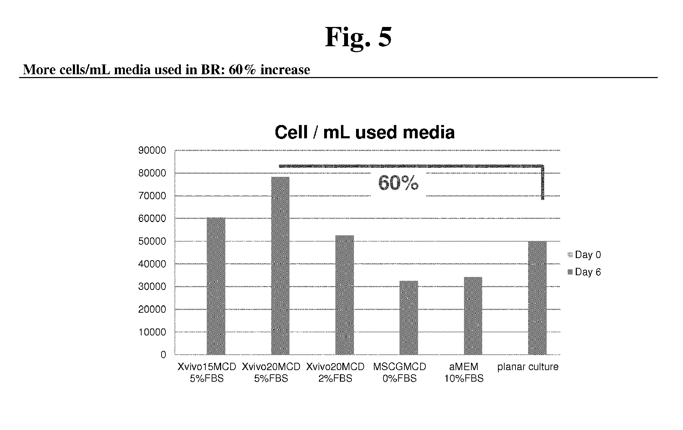

In addition culturing the stem cells in a bioreactor with microcarriers and the new media composition does not only allow serum-reduction but shows a 60% increase in cell yield per used ml media proving a real cost-effectiveness of the method using the new media composition. (Fig. 5).

### Example 3

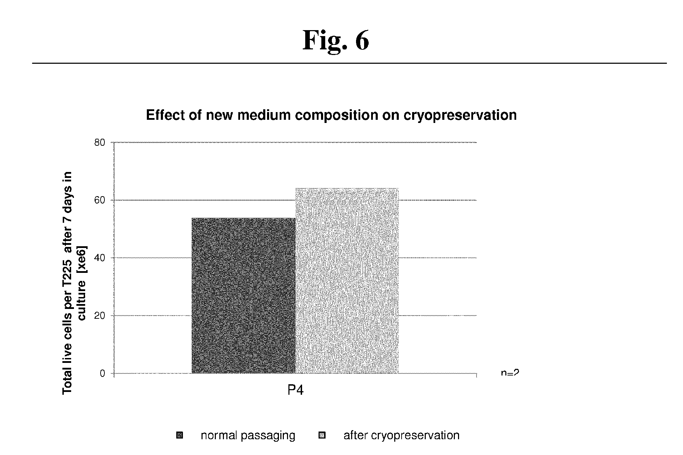

To test if cells retain their proliferative properties after cryopreservation, proliferation and viability of the cells was monitored after thawing and compared to cells that have been passaged. HMPC cells were cultured in X-VIVO20-CD SingleQuots 5 % serum. Cells were frozen in cryopreserving medium containing: Profreeze x2, 7.5% DMSO and X-VIVO20-CD SingleQuots medium replacing the normally used Alpha-Mem medium. (See Figure 6).

Cryopreservation of HMPCs in both Alpha-MEM- or X-VIVO - containing medium revealed good proliferation after thawing. In both media Alpha-Mem and X-VIVO20-CD Single Quots cells kept the same proliferation rate (>97 %) compared to cells that have been split. Cell growth and viability after freezing was comparable.

### Example 4

The new combination of media components also supports better growth of hBMMSCs on planar surfaces compared to the conditions commonly referred to as optimized standard conditions (DMEM 10% FBS).

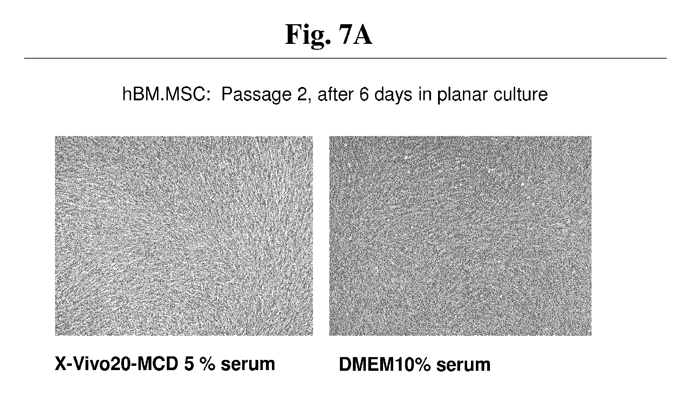

hBMMSC cells (Passage 2) after 6 days in planar culture grew better in X-VIVO20-CD Single Quots 5 % serum as compared to DMEM10% serum. See Figure 7A.

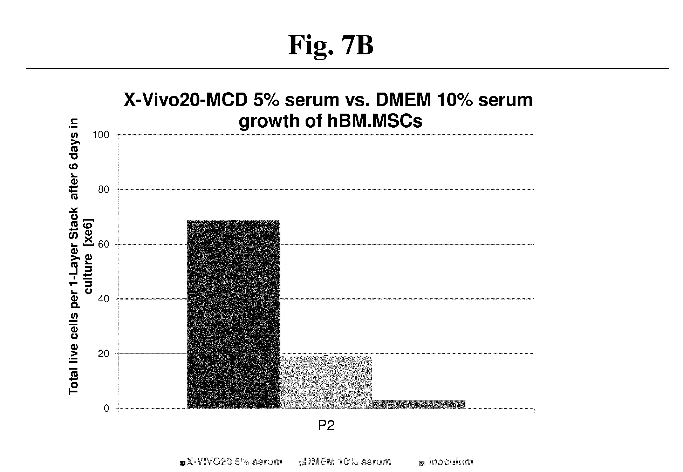

This is also depicted quantitatively as Total live cells per 1-Layer Stack after 6 days in culture [xe6] as show in Figure 7B.

## Claims

1. A cell culture medium comprising:
(a) a base medium; and
(b) optionally a media supplement;
wherein said base medium has a serum content of less than about 10% (v/v).

2. The cell culture medium of claim 1, wherein said base medium has a serum content of less than about 5% (v/v), preferably less than about 3% (v/v), particularly preferred less than about 2% (v/v).

3. The cell culture medium of claim lor 2, wherein the cell culture medium comprises sufficient elements to support the growth of an adult stem cell selected from the group consisting of: mesenchymal stem cells, induced pluripotent stem cells, progenitor cells, precursor cells.

4. The cell culture medium of any of claims 1-3, wherein the cell culture medium is a serum-free cell culture medium, an essentially serum free cell culture medium, or a serum reduced cell culture medium.

5. The cell culture medium of any of claims 1-4, wherein the base medium is selected from the group consisting of X-VIVO™ 10, X-VIVO™ 15, and X-VIVO™ 20.

6. The cell culture medium of any of claims 1-5 further comprising a microcarrier, and wherein said microcarrier is optionally selected from the group consisting of HILLEX II™, CYTODEX 3™, and plastic microcarrier.

7. The cell culture medium of any of claims 1-5, wherein the micocarrier is selected such that its surface structure and/or chemistry provide features missing in a particular medium.

8. The cell culture medium of claim 7, wherein the microcarrier is selected to provide an adhesion surface for an adult human mesenchymal stem cell.

9. The cell culture medium of any of claims 1-8, wherein the media supplement comprises at least one component selected from the group consisting of MSCGM-CD™ SingleQuots®, FGM-CD™ SingleQuot™, EGM-2 SingleQuot Kit Suppl. & Growth Factors, MesenCult™ Mesenchymal Stem Cell Stimulatory Supplements (Human), and StemPro® MSC SFM.

10. The cell culture medium of any of claims 1-9, wherein the media supplement is MSCGM-CD SingleQuots.

11. The cell culture medium of any of claims 1-10 comprising: a media base selected from the group consisting of X-VIVO 15 and X-VIVO 20; and optionally a media supplement selected from the group consisting of MSCGM-CD™ SingleQuots®, FGM-CD™ SingleQuot™, EGM-2 SingleQuot Kit Suppl. & Growth Factors, MesenCult™ Mesenchymal Stem Cell Stimulatory Supplements (Human), and StemPro® MSC SFM.

12. The cell culture medium of any of claims 1-10 comprising: a media base selected from the group consisting of X-VIVO 15 and X-VIVO 20; a microcarrier and optionally a media supplement selected from the group consisting of MSCGM-CD™ SingleQuots®, FGM-CD™ SingleQuot™, EGM-2 SingleQuot Kit Suppl. & Growth Factors, MesenCult™ Mesenchymal Stem Cell Stimulatory Supplements (Human), and StemPro® MSC SFM.

13. A method of expanding stem cells comprising growing the stem cells in a cell culture medium according to any of claims 1-12.

14. A stem cell culture obtained by a method according to claim 13.

15. A kit for growing stem cells, the kit comprising a base medium, and, optionally, a media supplement.
